# EUROPEAN PATENT SPECIFICATION

(11) **EP 0 596 165 B1**
(45) Date of publication and mention of the grant of the patent: **18.02.1998**
(21) Application number: 92310028.3
(22) Date of filing: 02.11.1992
(51) Int. Cl.: E21B 49/08, E21B 47/04, G01N 33/18, G01N 33/24

(54) **Well based sensor for detecting the presence of liquid hydrocarbons on groundwater**
Sensor zum Einbau in Brunnen zur Ueberwachung des Grundwassers vor Verunreinigungen durch Kohlenwasserstoffe
Sonde pour installation en puits piézométrique pour la détection d'hydrocarbures liquides surnageant les eaux souterraines

(43) Date of publication of application: 11.05.1994
(73) Proprietor: UNIVERSAL ENVIRONMENTAL TECHNOLOGIES LIMITED, Stowmarket, Suffolk IP14 2EN (GB)
(72) Inventor: Solomon, Harold, Nashua New Hampshire (US); Zamoida, Russell, Merrimack New Hampshire (US)
(74) Representative: W.P. Thompson & Co.

(56) References cited:
- DE-A- 2 559 233
- DE-A- 2 601 410
- DE-B- 1 296 832
- GB-A- 2 092 642
- US-A- 3 720 797
- US-A- 4 682 156
- US-A- 4 934 458
- US-A- 5 049 037

## Description

The present invention is concerned with a well-based sensor primarily for detecting the presence of liquid hydrocarbon pollutants, such as petroleum, diesel fuel, aviation fuels and certain other lighter than water, immiscible hydrocarbon liquids which may, due to accident or other reasons, pollute and float on underground water reserves.

The principal known method for the continuous monitoring of the underground watertable for the presence of floating hydrocarbon pollutants involves the use of a specially coated wire, the coating of which is soluble in these hydrocarbons. A double wire of this type is incorporated in an electrical circuit, together with a resistance, and is continuously monitored by an electrical resistivity sensor so that if degradation of the coating occurs the wires eventually touch and a change in resistance of the circuit is detected.

Other known sensor systems employ soluble polymers. One such system comprises a hose, or hoses, constructed of a hydrocarbon-soluble polymer. The hose is inserted into a well in such a way that it is partially submerged and connected to a supply of compressed air. If the hose material is dissolved by the presence of hydrocarbons, the drop in air pressure, which is constantly monitored by an air pressure sensor, is arranged to actuate an alarm. A problem with the latter system is that, like the abovedescribed coated wire system, where chafing of the wires can cause false alarms, the system must be constantly monitored and changes must be interpreted. Any leak in the air circuit may, for example, lead to a loss of air pressure and a subsequent false alarm.

Another known system uses a device which is held down by a weight attached to it by a hydrocarbon-soluble cable. If this cable dissolves, then the device floats upwards and sets off an alarm. The problem with the latter device is that very small thicknesses of hydrocarbon product may not come into direct contact with the cable, so it relies on there being either a thicker layer of hydrocarbons on the water surface, or sufficient concentration of miscible hydrocarbons in the water. It can therefore have a relatively slow response time.

A still further known system described in US-A- 4682152 comprises a hydrocarbon-soluble plastics rod which interconnects a mass or weight and a weighing assembly. The weighing assembly is operatively joined to an alarm indicator. When hydrocarbons are present, the plastics rod dissolves, causing movement of the weighing assembly and the alarm indicator so that a visual indication is provided that hydrocarbons are present.

Another known system described in DE-B-1296832 comprises a floating body disposed in a tube and carrying electrical contacts surrounded by a hydrocarbon-soluble material which normally prevents the electrical contacts from completing an electrical circuit. Should hydrocarbons be present in the tube the hydrocarbon-soluble material dissolves and the electrical circuit is completed so as to actuate an alarm.

A still further known system uses a floating apparatus incorporating a hydro-carbon soluble band which restrains the movement of spring-loaded switch. If the polymer is dissolved, the restraining pressure is released and the switch can activate an alarm circuit.

Such floating devices all have the disadvantages that they can "hang up" or catch and become caught on the perforated well casing during the natural fluctuations of the watertable. Also, although the switch may be carefully sealed, it represents a potential point of leakage.

A primary objective of the present invention is therefore the provision of a reliable and inexpensive means of positive detection of the presence of even very thin layers of petroleum, diesel fuel, aviation fuels and certain other lighter than water, immiscible hydrocarbon liquids which may, due to accident or other reasons, pollute and float on underground water reserves. Preferably, the system should have a means for the spontaneous and automatic alert of the water quality organisations and others concerned, both locally and at any remote location.

In accordance with the present invention, there is provided a sensor for indicating the presence of liquid hydrocarbons on or in groundwater, comprising a column of spacers which are made of a material which dissolves in or is structurally weakened by the liquid hydrocarbons concerned, and by a means of sensing the shortening of that column, should one or more of the spacers be dissolved, or partially dissolved, whereby to detect the presence of such hydrocarbon(s) in or on the groundwater.

Preferably, the sensor includes a primary support structure which simultaneously performs the functions of (a) separating and protecting from the groundwater/hydrocarbon(s) environment electrical components of an electrical signal generating part of the sensor forming said means for sensing shortening of said column of spacers (12), (b) supporting said column of spacers (12) and (c) acting as a primary component of the sensor for physical translation of the detection of the presence of liquid hydrocarbon(s) into actuation of the electrical signal-generating system.

Preferably, the primary support structure comprises a hollow spindle which supports the column of spacers.

Preferably, a first actuation float is disposed on the hollow spindle at the lower end of the column of spacers, the float carrying a means for actuating an electrical switching device disposed within the spindle.

In one embodiment, the actuating means carried by the float is a permanent magnet and the electrical switching device is a magnetic switch.

Advantageously, there is disposed within the spindle adjacent its lower extremity a second magnetic switch which responds to the float falling to a position indicative of the sensor being too high relative to the prevailing watertable level.

There may also be disposed within the spindle adjacent its upper extremity a third magnetic switch which responds to a second float, located on the spindle at the top end of said column of spacers, rising to a position indicative of the sensor being too low relative to the prevailing watertable level.

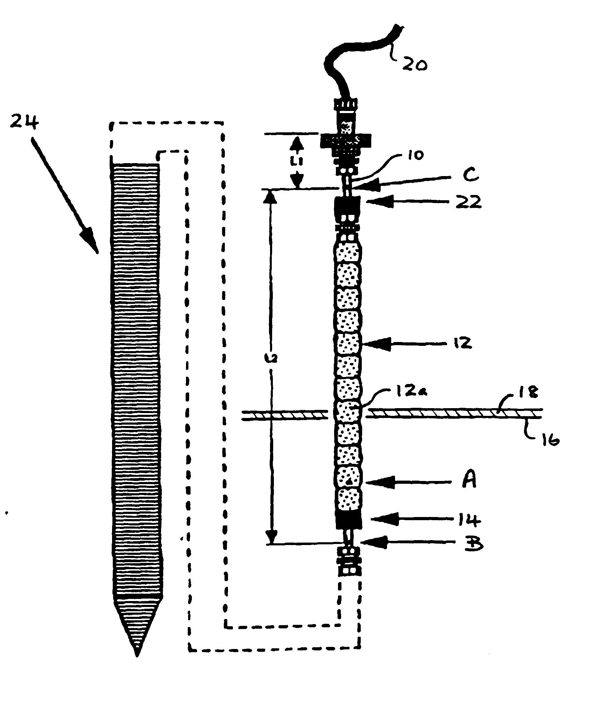

The invention is described further hereinafter, by way of example only, with reference to the accompanying drawing, which illustrates diagrammatically one embodiment of a groundwater pollution sensor in accordance with the present invention.

The illustrated sensor comprises a central hollow spindle 10, made of a material that will resist deterioration in water or liquid hydrocarbons. Disposed on the spindle 10 is a column of annular spacers 12 superposed on an annular float 14, the spindle 10 acting as a central core and guide for the column consisting of the float 14 and spacers 12, whose central aperture is dimensioned such that they can move freely along the spindle. The aforegoing assembly is adapted to be installed, in use, in a fixed position, vertically, in a small diameter well (not shown) in such a way that the column of polymer spacers 12 is partially submerged below the watertable. By way of example, a possible location of the watertable is indicated by the line 16.

The spacers 12 are made of a material which is soluble in petroleum, diesel fuels, aviation fuels and certain other liquid hydrocarbons, so that the presence of one of these hydrocarbons on top of the watertable, as indicated at 18, will result in the dissolving, or partial dissolving, of one of the spacers (in this case the spacer 12a). This has the result of causing a reduction in the length of the column and a physical movement upwards of the float 14 along the spindle 10.

Movement of the float 14 on the spindle is arranged to be detected by an electrical/electronic system disposed and encapsulated within the interior of the spindle 10. The latter system is therefore kept entirely separate from the external spacer/float arrangement. Any suitable means can be selected for translating the shortening of the column of spacers 12 embracing the central spindle 10 into an electrical signal. In a preferred embodiment, this is achieved using a permanent magnet which is embedded in the material of the annular float 14, and a magnetic switch (not visible in the drawing) disposed within the lower part of the spindle at a location A. The location A in the illustrated embodiment is above the starting position of the top edge of the float 14, whereby the system will be activated by one spacer being dissolved and the float 14 therefore rising by the height of that one spacer. The magnetic switch can be, for example, a reed switch or a solid state device such as a Hall effect switch. An output signal from the magnetic switch is led out of the top of the spindle by means of a cable 20 and via a sealed gland (not visible) so that the sealed integrity of the interior of the spindle will be maintained intact in use.

In an alternative movement detection system, the displacement of the float 14 on the spindle is arranged to result in the disturbance of an optical system mounted on the central spindle at the top of the column of spacers so as to cause deflection of a beam which is part of a photoelectric circuit.

In the illustrated embodiment, the float movement detection system is also extended to the operation of detecting excessive changes in the level of the watertable which would leave the detector inappropriately positioned relative to the watertable if not corrected. For this purpose, in addition to the magnetic switch located at position A, two further magnetic switches are located at B and C, respectively. Should the watertable drop too low, i.e. below the position occupied by the actuation float 14 in the drawing, the entire column of spacers 12 and the float 14 will descend on the spindle 10 and the magnet in the float 14 will actuate the second magnetic switch disposed within the spindle 10 adjacent its lower extremity at location B. On the other hand, an excessive rise in the watertable is detected by the provision of a second magnet (not shown) embedded in a further actuation float 22 mounted on the top of the column of spacers 12, this float 22 rising and actuating the third magnetic switch (not shown) located within the spindle close to its upper extremity at location C.

In most cases, it is appropriate to mount the spindle/spacer assembly within a separate, relatively rigid, protective screen 24 with a liquid-permeable perforated structure. This can have a pointed lower end to ease its insertion and location in small diameter wells without "hanging-up" or catching on joints or perforations in the wall casing.

It will be noted that the support structure of the abovedescribed system, namely the hollow spindle 10, simultaneously performs three different functions: (i) it acts as a sealed container, totally separating and isolating the electrical components from the physical and chemical elements of the sensor; (ii) it acts as the support for external means of detecting both the presence of liquid hydrocarbons and also incorrect positioning of the sensor relative to the watertable; and (iii) it acts as a primary component in the mechanism for the physical translation of the detection into the actuation of the electrical system.

The above described embodiment has the following practical features and advantages:
(a) The electrical system, which is encapsulated, is totally separated by a continuous barrier (the central spindle 10) which has no potential points of leakage. The electrical system is thus protected, and its isolation and safe electrical characteristics minimise any possibility of fire or explosion.
(b) The sensor can be chosen to operate with changes in level of the watertable without the problems of "hanging up" that are associated with the known floating devices; its column length can be manufactured to suit the normal variance in level of the watertable, and its correct position in the water is monitored by the two additional magnetic switches at locations B and C near the extremities of the central spindle, with a warning signal being given if the watertable should become relatively either too high or too low.
(c) The sensor's detection sensitivity can be adjusted according to different site conditions.
(d) The sensor can be replaced quickly.
(e) An unlimited number of sensors can be linked together in different configurations to suit different groundwater protection applications.
(f) The systems within which the sensors are incorporated not only report alarm information both locally and by telemetry, but also identify the cause of the alarm (sensor position too high or low/hydrocarbon presence alarm).
(g) The design of the system ensures that stress and breakage from outside influences are unlikely, especially as it is statically positioned in its own small diameter wall.
(h) The sensor system could be modified for use with heavier than water hydrocarbon pollutants.

## Claims

1. A sensor for indicating the presence of liquid hydrocarbons on or in groundwater including a sensing element which is soluble in liquid hydrocarbons, characterised by a column of spacers (12) which are made of material which dissolves in or is structurally weakened by the liquid hydrocarbon concerned, and by means of sensing the shortening of that column, should one or more of the spacers (12) be dissolved, or partially dissolved, whereby to detect the presence of such hydrocarbon(s) in or on the groundwater.

2. A sensor according to claim 1, wherein the column of spacers (12) is supported by a primary support structure in the form of a hollow spindle (10).

3. A sensor as claimed in claim 1, including a primary support structure (10) which simultaneously performs the functions of (a) separating and protecting from the groundwater/hydrocarbon(s) environment electrical components of an electrical signal generating part of the sensor forming said means for sensing shortening of said column of spacers (12), (b) supporting said column of spacers (12) and (c) acting as a primary component of the sensor for physical translation of the detection of the presence of liquid hydrocarbon(s) into actuation of the electrical signal-generating system.

4. A sensor according to claim 3, wherein the primary support structure comprises a hollow spindle (10) which supports the column of spacers (12).

5. A sensor according to claim 2 or 4, wherein a first actuation float (14) is disposed on the hollow spindle (10) at the lower end of the column of spacers (12), the float (14) carrying a means for actuating an electrical switching device disposed within the spindle.

6. A sensor according to claim 5, wherein the actuating means carried by the float (14) is a permanent magnet and the electrical switching device is a magnetic switch.

7. A sensor according to claim 6, wherein there is disposed within the spindle (10) adjacent its lower extremity a second magnetic switch which responds to the float (14) falling to a position indicative of the sensor being too high relative to the prevailing watertable level.

8. A sensor according to claim 7, wherein there is disposed within the spindle (10) adjacent its upper extremity a third magnetic switch which responds to a second float (22), located on the spindle (10) at the top end of said column of spacers (12), rising to a position indicative of the sensor being too low relative to the prevailing watertable level.

9. A sensor according to any of claims 1 to 8, wherein the spacers (12) are of annular configuration.

10. A sensor according to any of claims 2 to 9, wherein the primary support structure (10) and column of spacers (12) are embraced by a rigid screen (24) of liquid-permeable perforated structure.

## Patentansprüche

1. Sensor zum Anzeigen der Anwesenheit von flüssigen Kohlenwasserstoffen auf dem oder im Grundwasser, umfassend ein Sensorelement, das in flüssigen Kohlenwasserstoffen löslich ist, gekennzeichnet durch eine Säule von Abstandshaltern (12), die aus einem Material hergestellt sind, das sich in den betroffenen flüssigen Kohlenwasserstoffen auflöst oder von diesen strukturell geschwächt wird, und durch ein Mittel zum Erfassen des Kürzerwerdens dieser Säule, wenn sich ein oder mehrere der Abstandshalter (12) auflösen oder teilweise auflösen, wodurch die Anwesenheit (eines) solchen/r Kohlenwasserstoffe(s) im oder auf dem Grundwasser erfaßt wird.

2. Sensor nach Anspruch 1, bei dem die Säule aus Abstandshaltern (12) von einer primären Tragstruktur in der Form einer Hohlspindel (10) getragen wird.

3. Sensor nach Anspruch 1, umfassend eine primäre Tragstruktur (10), die gleichzeitig die folgenden Funktionen erfüllt: (a) Trennen und Schützen elektrischer Komponenten eines elektrische Signale generierenden Teils des Sensors, der das genannte Mittel zum Erfassen des Kürzerwerdens der genannten Säule von Abstandshaltern (12) bildet, von/vor der Grundwasser/Kohlenwasserstoff-Umgebung, (b) Tragen der genannten Säule von Abstandshaltern (12), und (c) Wirkung als Hauptkomponente des Sensors für eine physikalische Umsetzung des Erfassens der Anwesenheit (des) flüssigen/r Kohlenwasserstoffe(s) in eine Betätigung des elektrische Signale generierenden Systems.

4. Sensor nach Anspruch 3, bei dem die primäre Tragstruktur eine Hohlspindel (10) umfaßt, die die Säule von Abstandshaltern (12) trägt.

5. Sensor nach Anspruch 2 oder 4, bei dem ein erster Betätigungsschwimmer (14) auf der Hohlspindel (10) am unteren Ende der Säule von Abstandshaltern (12) angeordnet ist, wobei der Schwimmer (14) ein Mittel zum Betätigen einer in der Spindel befindlichen elektrischen Schaltvorrichtung trägt.

6. Sensor nach Anspruch 5, bei dem das von dem Schwimmer (14) getragene Betätigungsmittel ein Dauermagnet und die elektrische Schaltvorrichtung ein Magnetschalter ist.

7. Sensor nach Anspruch 6, bei dem in der Spindel (10) an seiner unteren Spitze ein zweiter Magnetschalter angeordnet ist, der anspricht, wenn der Schwimmer (14) bis auf eine Position fällt, die anzeigt, daß der Sensor relativ zum vorherrschenden Grundwasserspiegel zu hoch ist.

8. Sensor nach Anspruch 7, bei dem in der Spindel (10) neben dem oberen Ende ein dritter Magnetschalter angeordnet ist, der auf einen zweiten Schwimmer (22) anspricht, der sich an der Spindel (10) an der oberen Spitze der genannten Säule von Abstandshaltern (12) befindet und der bis zu einer Position ansteigt, die anzeigt, daß der Sensor relativ zum vorherrschenden Grundwasserspiegel zu niedrig ist.

9. Sensor nach einem der Ansprüche 1 bis 8, bei dem die Abstandshalter (12) ringförmig ausgestaltet sind.

10. Sensor nach einem der Ansprüche 2 bis 9, bei dem die primäre Tragstruktur (10) und die Säule von Abstandshaltern (12) von einer starren Abschirmung (24) aus einer flüssigkeitsdurchlässigen perforierten Struktur umgeben sind.

## Revendications

1. Sonde pour indiquer la présence d'hydrocarbures liquides sur ou dans des eaux souterraines comprenant un élément de détection qui est soluble dans les hydrocarbures liquides, caractérisée par une colonne d'entretoises (12) qui sont faites d'un matériau qui est dissous ou structurellement affaibli par l'hydrocarbure liquide concerné, et par des moyens de détection du raccourcissement de cette colonne si une ou plusieurs des entretoises (12) est dissoute, ou partiellement dissoute, de manière à détecter la présence de ce ou ces hydrocarbure(s) dans ou sur les eaux souterraines.

2. Sonde selon la revendication 1, dans laquelle la colonne d'entretoises (12) est supportée par une structure de support principale sous la forme d'un arbre creux (10).

3. Sonde selon la revendication 1, comprenant une structure de support principale (10) qui remplit simultanément les fonctions visant à (a) séparer et protéger contre l'environnement d'eaux souterraines/ hydrocarbures des composants électriques d'une partie de génération de signal électrique de la sonde formant lesdits moyens de détection du raccourcissement de ladite colonne d'entretoises (12), (b) supporter ladite colonne d'entretoises (12) et (c) servir de composant principal de la sonde assurant le transfert physique de la détection de la présence d'hydrocarbure(s) liquide(s) en actionnement du système de génération de signal électrique.

4. Sonde selon la revendication 3, dans laquelle la structure de support principale comprend un arbre creux (10) qui supporte la colonne d'entretoises (12).

5. Sonde selon la revendication 2 ou 4, dans laquelle un premier flotteur d'actionnement (14) est disposé sur l'arbre creux (10) à l'extrémité inférieure de la colonne d'entretoises (12), le flotteur (14) portant des moyens pour actionner un dispositif de commutation électrique disposé dans l'arbre.

6. Sonde selon la revendication 5, dans laquelle les moyens d'actionnement portés par le flotteur (14) sont constitués par un aiment permanent et le dispositif de commutation électrique est un commutateur magnétique.

7. Sonde selon la revendication 6, dans laquelle est disposé dans l'arbre (10), à proximité de son extrémité inférieure, un deuxième commutateur magnétique qui réagit à la chute du flotteur (14) à une position indiquant que la sonde est trop haute par rapport au niveau de la nappe phréatique prévalent.

8. Sonde selon la revendication 7, dans laquelle est disposé dans l'arbre (10), à proximité de son extrémité supérieure, un troisième commutateur magnétique qui réagit à la montée d'un deuxième flotteur (22), situé sur l'arbre (10) à l'extrémité supérieure de ladite colonne d'entretoises (12), à une position indiquant que la sonde est trop basse par rapport au niveau de la nappe phréatique prévalent.

9. Sonde selon l'une quelconque des revendications 1 à 8, dans laquelle les entretoises (12) ont une configuration annulaire.

10. Sonde selon l'une quelconque des revendications 2 à 9, dans laquelle la structure de support principale (10) et la colonne d'entretoises (12) sont serrées dans un écran rigide (24) ayant une structure perforée perméable aux liquides.
